# EUROPEAN PATENT APPLICATION

(11) **EP 3 913 637 A1**
(43) Date of publication of application: **24.11.2021**
(21) Application number: 20175982.6
(22) Date of filing: 22.05.2020
(51) Int. Cl.: G16H 30/40, G16H 40/67, G16H 50/20, G16H 20/10

(54) **SYSTEM AND METHOD FOR MONITORING PATIENT DATA AND CONTROL TREATMENT**

(71) Applicant: Infomining Co., Ltd., Gyeonggi-do 14548 (KR)
(72) Inventor: LEE, Jae Yong, 14585 Bucheon-si (KR)
(74) Representative: Kurig, Thomas

(57) **Abstract**

Disclosed is a hospital management system including a communication unit for transmitting and receiving data, a memory for storing at least one instruction, and at least one processor for executing the at least one instruction stored in the memory. The at least one processor controls the communication unit to receive biometrics of a patient in real time, determines a disease symptom variation of the patient based on an examination result and the biometrics, wherein the examination result includes at least one of CT, Xray, MRI results, a body temperature, a pulse, a blood pressure, an oxygen saturation, an electrocardiogram, or a blood sugar level of the patient, controls the communication unit to transmit at least one of the examination result, the biometrics, or the disease symptom variation to a hospital electronic medical record system and a medical staff device, and receives prescription information from a medical staff, and performs prescription corresponding to the prescription information for the patient.

## Description

### BACKGROUND

Embodiments of the inventive concept described herein relate to a hospital management system and a control method thereof. More specifically, embodiments of the inventive concept described herein relate to a hospital management system and a control method thereof, in which change in a disease state is determined based on an examination result and biometrics of a patient, and a medical staff performs, in a non-contact manner or remotely, diagnosis and prescription of the patient based on the determination result.

In an environment of a fixed diagnosis time, a limited number of medical staffs, and many patient, a conventional medical system may not provide high-quality medical services. Therefore, in order to provide the high quality medical services, medical services in which diagnosis and prescription are made while remotely monitoring a patient in real time are required.

Moreover, recently, highly contagious diseases are prevalent worldwide. The highly contagious disease may be transmitted not only to a general public, but also to a medical staff who treats patient, resulting in a shortage of the medical staff and in difficulty in rapid eradication of the disease. Therefore, there is a need to provide a medical service for diagnosis, prescription, and treatment of a patient in a non-contact manner or remotely.

### SUMMARY

Embodiments of the inventive concept provide a hospital management system and a control method thereof. Specifically, embodiments of the inventive concept provide a hospital management system and a control method thereof in which change in a disease state is determined based on an examination result and biometrics of a patient, and a medical staff performs, in a non-contact manner or remotely, diagnosis and prescription of the patient based on the determination result.

According to an exemplary embodiment, a hospital management system includes a communication unit for transmitting and receiving data, a memory for storing at least one instruction, and at least one processor for executing the at least one instruction stored in the memory. The at least one processor controls the communication unit to receive biometrics of a patient in real time, determines a disease symptom variation of the patient based on an examination result and the biometrics, wherein the examination result includes at least one of CT, Xray, MRI results, a body temperature, a pulse, a blood pressure, an oxygen saturation, an electrocardiogram, or a blood sugar level of the patient, controls the communication unit to transmit at least one of the examination result, the biometrics, or the disease symptom variation to a hospital electronic medical record system and a medical staff device, and receives prescription information from a medical staff, and performs prescription corresponding to the prescription information for the patient.

The biometrics may include at least one of a body temperature, a pulse, a blood pressure, an oxygen saturation, an electrocardiogram, and a blood sugar level of the patient, and body information corresponding to voice information input from the patient.

At least one of the body temperature, the pulse, the blood pressure, the oxygen saturation, the electrocardiogram, or the blood sugar level among the biometrics may be checked in real time by a wearable device of the patient, wherein the at least one processor may control the communication unit to receive the biometrics checked by the wearable device in real time.

The at least one processor may change a data format of the examination result and the disease symptom variation to a data format of data stored in the hospital electronic medical record system and transmit the changed data format of the examination result and the disease symptom variation to the hospital electronic medical record system.

The disease symptom variation of the patient may include a variation of the biometrics corresponding to a disease of the patient. The at least one processor may transmit an alert signal to the hospital electronic medical record system and the medical staff device when the disease symptom variation exceeds a preset threshold.

The prescription information may be received remotely from the medical staff device. The prescription information may include at least one of a type of a drug to be administered to the patient, a dosage of the drug, or an administration period of the drug.

The at least one processor may provide the patient with an instruction to administer a drug delivered to the patient by the medical staff in a non-contact manner or based on remote prescription.

The examination result, the biometrics, and the disease symptom variation of the patient may act as training data for machine learning. The at least one processor may learn the training data to determine at least one of a likelihood of symptom change of a disease of the patient, whether the disease is confirmed, or whether the disease has been cured.

The medical staff device may include at least one of an electronic device of a nursing station, or an electronic device of doctor.

According to an exemplary embodiment, a method for controlling a hospital management system includes receiving biometrics of a patient in real time, determining a disease symptom variation of the patient based on an examination result and the biometrics, wherein the examination result includes at least one of CT, Xray, MRI results, a body temperature, a pulse, a blood pressure, an oxygen saturation, an electrocardiogram, or a blood sugar level of the patient, transmitting at least one of the examination result, or the disease symptom variation to a hospital electronic medical record system and a medical staff device, and receiving prescription information from a medical staff, and performing prescription corresponding to the prescription information for the patient.

The disease symptom variation of the patient may include a variation of the biometrics corresponding to a disease of the patient. The determining of the disease symptom variation of the patient may include transmitting an alert signal to the hospital electronic medical record system and the medical staff device when the disease symptom variation exceeds a preset threshold.

### BRIEF DESCRIPTION OF THE FIGURES

The above and other objects and features will become apparent from the following description with reference to the following figures, wherein like reference numerals refer to like parts throughout the various figures unless otherwise specified, and wherein:
FIG. 1 is a block diagram showing a hospital management system according to an embodiment of the inventive concept;
FIG. 2 is a schematic diagram illustrating an interaction relationship between a hospital management system, a medical staff device and a hospital electronic medical record system according to an embodiment of the inventive concept;
FIG. 3 is a view showing an example of diagnosis and prescription of a patient according to an embodiment of the inventive concept;
FIG. 4 is a view showing an example of diagnosis and prescription of a patient according to an embodiment of the inventive concept;
FIG. 5 is a flowchart showing a method of controlling a hospital management system according to an embodiment of the inventive concept; and
FIG. 6 is a flowchart showing a method of controlling a hospital management system according to an embodiment of the inventive concept.

### DETAILED DESCRIPTION

Advantages and features of the inventive concept, and methods of achieving them will become apparent with reference to embodiments described below in detail in conjunction with the accompanying drawings. However, the inventive concept is not limited to the embodiments disclosed below, but may be implemented in various forms. The present embodiments are provided to merely complete the disclosure of the inventive concept, and to inform merely fully those skilled in the art of the inventive concept of the scope of the inventive concept. The inventive concept is only defined by the scope of the claims. Like reference numerals refer to like elements throughout the disclosure.

The terminology used herein is for the purpose of describing the embodiments only and is not intended to limit the inventive concept. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof. As used herein, the term "and/or" includes any and all combinations of one or more of the associated listed items. Expression such as "at least one of" when preceding a list of elements may modify the entire list of elements and may not modify the individual elements of the list.

The word "exemplary" is used herein to mean "used as an illustration or an exemplification". Any "exemplary" embodiment as described herein should not necessarily be construed as being preferred or having advantages over other embodiments.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

A deep neural network (DNN) according to embodiments of the inventive concept means a system or network that builds at least one layer in at least one computer and performs determinations based on a plurality of data. For example, the deep neural network may be implemented as a set of layers including a convolutional pooling layer, a locally-connected layer, and a fully-connected layer. The convolutional pooling layer or the locally-connected layer may be configured to extract features in an image. The fully-connected layer may determine a correlation between the features of the image. In some embodiments, an overall structure of the deep neural network may be constructed such that the convolutional pooling layer is connected to the locally-connected layer which in turn is connected to the fully-connected layer. The deep neural network may include various determination criteria, that is, parameters, and may add new determination criteria, that is, new parameters, via analysis of an input image.

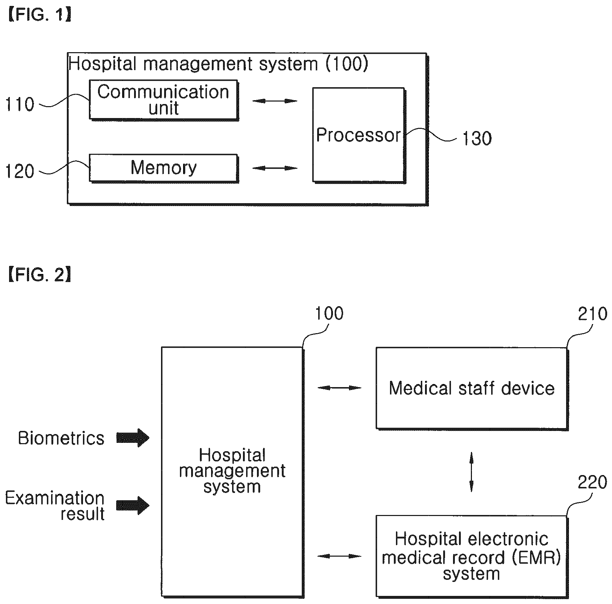

The deep neural network according to embodiments of the inventive concept has a structure referred to as a convolutional neural network suitable for image analysis, as shown in FIG. 2. The deep neural network has a structure in which a feature extraction layer that autonomously learns a feature with the greatest discriminative power from given image data is integrated with a prediction layer that learns a prediction model to produce the highest prediction performance based on the extracted feature.

The feature extraction layer has a structure in which a convolution layer for applying a plurality of filters to each region of the image to create a feature map, and a pooling layer for pooling feature maps spatially to extract a feature invariant relative to change in a position or a rotation are repeated alternately with each other multiple times. Thus, the feature extraction layer may extract various levels of features from low-level features such as points, lines, and surfaces to complex and meaningful high-level features.

The convolution layer applies a non-linear activation function to a dot product between a filter and a local receptive field for each patch of an input image to obtain the feature map. Compared to other network architectures, a convolutional neural network (CNN) uses a filter having sparse connectivity and shared weights. This connection structure reduces the number of models to be learned, and realizes efficient learning via a backpropagation algorithm, resulting in improved prediction performance.

The pooling layer (or a sub-sampling layer) creates a new feature map by utilizing local information of the feature map obtained from the previous convolution layer. In general, the feature map newly created by the pooling layer is reduced to a smaller size than a size of an original feature map. A typical pooling method includes a max pooling method which selects a maximum value of a corresponding region in the feature map, and an average pooling method which calculates an average of a corresponding region in the feature map. The feature map of the pooling layer is generally less affected by a location of any structure or pattern in the input image than a feature map of the previous layer is. That is, the pooling layer may extract a feature that is more robust to a regional change such as noise or distortion in the input image or the previous feature map. This may play an important role in classification performance. Another role of the pooling layer is to allow a feature of a wider region to be reflected as a layer goes up to a top learning layer in a deep structure. Thus, the features may be created such that as the feature extraction layers are accumulated one on top of another, a lower layer reflects a local feature and a higher layer reflects an abstract feature of an entire image.

In this way, a feature that was finally extracted via repetition of the convolution layer and the pooling layer may be combined with a classification model such as MLP (Multi-Layer Perception) or SVM (Support Vector Machine) in a form of the fully-connected layer and thus may be used for learning and prediction of the classification model.

However, a structure of the deep neural network according to the embodiments of the inventive concept is not limited thereto. Rather, neural networks of various structures may be employed.

Hereinafter, embodiments of the inventive concept will be described in detail with reference to the accompanying drawings.

FIG. 1 is a block diagram showing a hospital management system 100 according to an embodiment of the inventive concept. The hospital management system 100 may include a communication unit 110, a memory 120 and a processor 130.

According to an embodiment of the inventive concept, the communication unit 110 may receive data transmitted to the hospital management system 100 and transmit data stored in the memory 120 or data processed by the processor 130 to an external device. Moreover, the communication unit 110 of the inventive concept may perform communication with various types of external devices according to various types of communication protocols. The communication unit 110 may include at least one of a WiFi chip, a Bluetooth chip, a wireless communication chip, and an NFC chip.

The Wi-Fi chip and the Bluetooth chip may perform communication using a WiFi protocol and a Bluetooth protocol, respectively. When using the Wi-Fi chip or the Bluetooth chip, various connection information such as an SSID and a session key may be first transmitted and received, and then, communication connection may be established using the connection information to transmit and receive various information. The wireless communication chip means a chip that performs communication according to various communication standards such as IEEE, Zigbee, 3G (3rd Generation), 3GPP (3rd Generation Partnership Project), and LTE (Long Term Evolution). The NFC chip means a chip operating in an NFC (Near Field Communication) protocol using a 13.56 MHz band among various RF-ID frequency bands such as 135 kHz, 13.56 MHz, 433 MHz, 860 to 960 MHz, and 2.45 GHz.

The memory 120 of the inventive concept is a local storage medium that may store data received by the communication unit 110 and data processed by the processor 130. When necessary, the communication unit 110 and the processor 130 may use the data stored in the memory 120. Moreover, the memory 120 of the inventive concept may store patient examination results, patient biometrics, and instructions to enable the processor 130 to operate.

Moreover, the data stored in the memory 120 may be used to store a learning model of the hospital management system 100 to identify a disease of a patient and whether the disease has been removed. Furthermore, the memory 120 of the inventive concept may be embodied as a writable non-volatile memory (e.g., writable ROM) in which data is maintained when power supplied to the hospital management system 100 is cut off, and data change is reflected. That is, the memory 120 may be embodied as one of a flash memory or EPROM or EEPROM. Herein, an example in which all instruction information are stored in the single memory 120 is set forth for convenience of description. However, the present disclosure is not limited thereto. The hospital management system 100 may have a plurality of memories.

According to an embodiment of the inventive concept, the processor 130 may control the communication unit 110 to receive the biometrics of the patient in real time, and may determine disease symptom variation of the patient based on the biometrics and the examination results thereof. The processor 130 of the inventive concept may perform all operations and steps of the hospital management system 100 as described below.

In accordance with the inventive concept, the patient's biometrics may include at least one of a body temperature, a pulse, a blood pressure, an oxygen saturation, an electrocardiogram, a blood sugar level of the patient, and body information corresponding to voice information input from the patient.

In accordance with the inventive concept, the examination result is the result of a basic examination performed when the patient is first admitted to the hospital. The examination result may include at least one of CT, Xray, MRI results, a body temperature, a pulse, a blood pressure, an oxygen saturation, an electrocardiogram, and a blood sugar level of the patient.

In accordance with the inventive concept, the patient's disease symptom variation may be a variation of the biometrics corresponding to the patient's disease. For example, for a patient suffering from COVID-19, the body temperature may be the biometrics corresponding to the disease. In another example, for a patient suffering from a common cold, the biometrics associated with the disease may be the body temperature and a white blood cell count. Therefore, in the patient suffering from COVID-19, a variation in the body temperature may be the disease symptom variation. In the patient with the cold, a variation in the body temperature and a variation in the white blood cell count may be the disease symptom variation.

Because the body temperatures, the pulses, the blood pressures, and the white blood cell counts of the patients may not be equal to each other, the hospital management system 100 determines the patient's disease symptom variation based on the patient's biometrics and the patient's examination results received in real time. For example, when the patient has a relatively low blood pressure compared to other people, the hospital management system 100 may compare a blood pressure according to the patient's examination result with a blood pressure received in real time during hospitalization and determine a patient's disease symptom variation based on the comparison result.

According to an embodiment of the inventive concept, the hospital management system 100 may determine the patient's disease symptom variation, and then may transfer at least one of the patient's examination result and the patient's disease symptom variation to the medical staff device and the hospital electronic medical record system via the communication unit 110. In this case, a data format of the patient's examination record and disease symptom variation data may be different from a format of data stored in the hospital electronic medical record system. Thus, the hospital management system 100 may change the data format of the patient's examination result and disease symptom variation to the data format of the data stored in the hospital electronic medical record system and then transmit the changed data format of the patient's examination result and disease symptom variation thereto. The changing of the data format may be to change a format of a stored file, or may be to compress or split a file to reduce a size of the file.

According to an embodiment of the inventive concept, the medical staff device may include an electronic device used by a nursing station and a doctor in a hospital. Therefore, when at least one of the patient's examination result and disease symptom variation is sent to the electronic device, the doctor may check the transmitted data via a dedicated application to the hospital where he/she works. Thus, the medical staff may check the patient's disease symptom variation in real time, and may pre-check abnormal biometrics based on the examination result.

After checking the patient's disease symptom variation, the medical staff may transmit prescription information suitable for the patient's current situation to the hospital management system 100. When the prescription information is transmitted to the hospital management system 100, the hospital management system 100 may perform a prescription corresponding to the transmitted prescription information. For example, when the hospital management system 100 is connected to an AI (artificial intelligence) speaker in a patient room where the patient is hospitalized, the hospital management system 100 may provide the prescription information from the medical staff to the patient through the AI speaker. Specifically, when the patient's current disease symptom status requires an injection of 2 ml of insulin, the doctor may send the prescription information indicating that 2 mL of insulin should be injected to the hospital management system 100 through his/her electronic device. Then, the hospital management system 100 may instruct the patient to inject 2 mL of insulin among drugs to be ready to be administered thereto through the AI speaker.

FIG. 2 is a schematic diagram for illustrating an interaction relationship between the hospital management system 100, a medical staff device 210 and a hospital electronic medical record system 220 according to an embodiment of the inventive concept. Referring to FIG. 2, the hospital management system informs the medical staff of the patient's current condition and explains the process of the medical staffs prescription.

The hospital electronic medical record (EMR) system 220 is configured to computationally process a medical record among hospital diagnosis support records and has an effect of promptly processing a task in the hospital and reducing a manpower and a cost, and quickly transmits and utilizes records. Further, the hospital EMR system 220 has an effect of improving a service, such as shortening a patient's waiting time. Thus, the hospital EMR system 220 is currently employed and operated in most of hospitals. In accordance with the inventive concept, in order to store all medical records of the hospital, information transmitted by the hospital management system 100 and information transmitted by the medical staff device 210 may be stored in the hospital electronic medical record system 220.

According to an embodiment of the inventive concept, the patient is subjected to a basic examination when being hospitalized. In this case, the examination result may include CT, Xray, MRI results, a body temperature, pulse, a blood pressure, an oxygen saturation, an electrocardiogram, and a blood sugar level of the patient. The examination result may be transmitted to the hospital management system 100 and then may be transmitted via the hospital management system 100 to the medical staff device 210 and the hospital electronic medical record system 220. When the patient being tested is not first admitted to the hospital, the patient's medical records, for example, examination results are all stored in the hospital electronic medical record system 220. Thus, the hospital management system 100 and the medical staff device 210 may receive the patient's previous medical records from the hospital electronic medical record system 220 when necessary.

When the patient's examination is complete, the hospital management system 100 may receive the examination results, and biometrics after the patient is admitted, in real time. The patient may be first hospitalized in the hospital or the patient was previously hospitalized therein. Thus, a transmission order of the examination results and the biometrics is not important and they may be transmitted simultaneously.

The patient's biometrics may be transmitted in real time and may include a body temperature, a pulse, a blood pressure, an oxygen saturation, an electrocardiogram, a blood sugar level of the patient, or body information corresponding to voice information input from the patient. In this case, the body temperature, the pulse, the blood pressure, the oxygen saturation, the electrocardiogram, and the blood sugar level may be transmitted in real time to the hospital management system 100 through a wearable device (not shown) provided by the hospital. Specifically, the wearable device may check the biometrics of the patient in real time using an EMG sensor (electromyogram sensor) and transmit the checked biometrics to the hospital management system 100. However, the patient's biometrics are not necessarily checked only through the wearable device. Rather, the biometrics may be checked and transmitted through other series of electronic devices provided by the hospital.

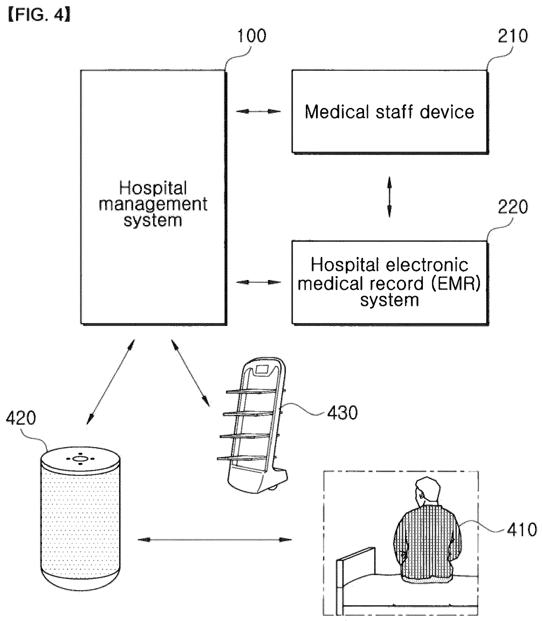

According to an embodiment of the inventive concept, when the AI speaker is installed in the patient room where the patient is hospitalized, the AI speaker may be connected to the hospital management system 100 over a network. Therefore, the patient may inform the medical staff of his/her body condition in a voice manner. Specifically, when the patient's voice information is transmitted to the hospital management system 100 through the AI speaker, the hospital management system 100 may determine the body information corresponding to the voice information of the patient and may send the body information to the medical staff device 210 and the hospital electronic medical record system 220. In accordance with the inventive concept, the body information corresponding to the voice information of the patient may be composed of terms related to the body as extracted among the voice information uttered by the patient and may indicate a current body state of the patient from a medical point of view. For example, when the patient utters "I have an ache at a lower stomach", the voice information "I have an ache at a lower stomachache" may be transmitted to the hospital management system 100 through the AI speaker. In this case, the hospital management system 100 may determine "lower abdominal pain" as the patient's current body information, may use the body information as the biometrics, and may transmit the biometrics to the medical staff device 210 and the hospital electronic medical record system 220. Providing the voice information through the AI speaker will be described in detail with reference to FIG. 4.

Referring back to FIG. 2, when the biometrics and the examination results are transmitted, the hospital management system 100 may determine the patient's disease symptom variation based on the biometrics and the examination results. The patient's disease symptom variation may refer to a variation in biometrics corresponding to the patient's disease. Thus, the hospital management system 100 may determine the variation in the biometrics related to a main symptom of the patient's disease. For example, in the patient suffering from COVID-19, the body temperature may be the biometrics corresponding to the disease. Moreover, COVID-19 may be accompanied by a symptom of pneumonia. Thus, the white blood cell count may be the biometrics corresponding to the disease. Therefore, in the patient suffering from COVID-19, the disease symptom variation may include a variation in the body temperature or a variation in the white blood cell count.

When the determination of the disease symptom variation is completed, the hospital management system 100 may transmit the determined disease symptom variation to the medical staff device 210 and the hospital electronic medical record system 220. In this case, the examination result, the biometrics and the disease symptom variation of the patient are different information from each other. For this reason, the hospital management system 100 may simultaneously transmit the examination result, the biometrics and the disease symptom variation in a single data packet or may transmit them sequentially and individually over time.

According to an embodiment of the inventive concept, when the hospital management system 100 determines that the disease symptom variation exceeds a preset threshold, the hospital management system 100 may transmit an alert signal to the medical staff device 210 and the hospital electronic medical record system 220. Different diseases may have different biometrics or disease symptom vibrations, based on which a patient's critical condition is determined. Therefore, the hospital electronic medical record system 220 may set different thresholds corresponding to different diseases from which the patient currently suffers or which are conventionally known. In this connection, the threshold may refer to a disease symptom variation, based on which the patient's critical condition is determined. In this case, the threshold may be pre-stored by the medical staff or may be stored in real time by the medical staff. For example, when a body temperature variation of the patient suffering from COVID-19 suddenly exceeds 10 degrees, the hospital management system 100 may transmit the alert signal to the medical staff device 210 and the hospital electronic medical record system 220. In accordance with the inventive concept, the alert signal may be transmitted in a text form, or may be sound information. In the latter case, when the alert signal is transmitted to the medical staff device 210, a sound occurs.

According to an embodiment of the inventive concept, when the disease symptom variation is transmitted to the medical staff device 210, the doctor may make a prescription to alleviate a current symptom of the patient. In this case, when necessary, the doctor may refer to the patient's previous examination result stored in the hospital electronic medical record system 220, in addition to the disease symptom variation, and may refer to a type or a symptoms of the patient's previous illness.

When the doctor makes the prescription, the prescription information may be transmitted to the hospital electronic medical record system 220 and the hospital management system 100. In this case, the hospital management system 100 may perform prescription corresponding to the received prescription information.

In accordance with the inventive concept, the prescription information may include a type of a drug to be administered to the patient, a dosage thereof, or an administration period thereof.

According to embodiments of the inventive concept, a manner in which the hospital management system 100 performs the prescription corresponding to the received prescription information may vary. In an embodiment, as the hospital management system 100 requests a drug delivery robot to deliver a specific drug to a patient according to the prescription information as received, the drug delivery robot may deliver the drug corresponding to the prescription information to the patient room where the patient is hospitalized. In another embodiment, when the medical staff delivers the drug to the patient, the hospital management system 100 may remotely control an entrance door of the patient room. Specifically, when there are multiple entrance doors to the patient room where the patient is hospitalized (e.g., as in a negative-pressure patient room), the medical staff puts the drug to be delivered to the patient at a location between a first entrance door and a second entrance door, and then the hospital management system 100 opens the second entrance door while locking the first entrance door in direct contact with the patient. Subsequently, when the medical staff has finished delivering the drug, the first entrance door may be opened and the second entrance door may be closed at the same time. Thus, according to embodiments of the inventive concept, after the patient is hospitalized, the medical staff may diagnose or prescribe the patient remotely or in a non-contact manner with the patient.

According to an embodiment of the inventive concept, a scheme in which the hospital management system 100 performs the prescription corresponding to the received prescription information may include a scheme of providing an instruction corresponding to the received prescription information to the patient. Specifically, the hospital management system 100 may inform the patient of a type of a drug, an administration method, a dosage, and an administration period thereof corresponding to the prescription information. For example, when the medical staff puts the drug at the location between the first entrance door and the second entrance door in a non-contact manner, the hospital management system 100 may inform the patient of an instruction for administering the drug using a display device (not shown) or the AI speaker in the patient room.

According to an embodiment of the inventive concept, the examination result, the biometrics, and the disease symptom variation of the patient may act as training data for machine learning. The hospital management system 100 may learn the training data using the deep neural network. Therefore, as the hospital management system 100 learns the training data, the hospital management system 100 may determine whether a disease of the hospitalized patient is confirmed or whether the disease has been cured.

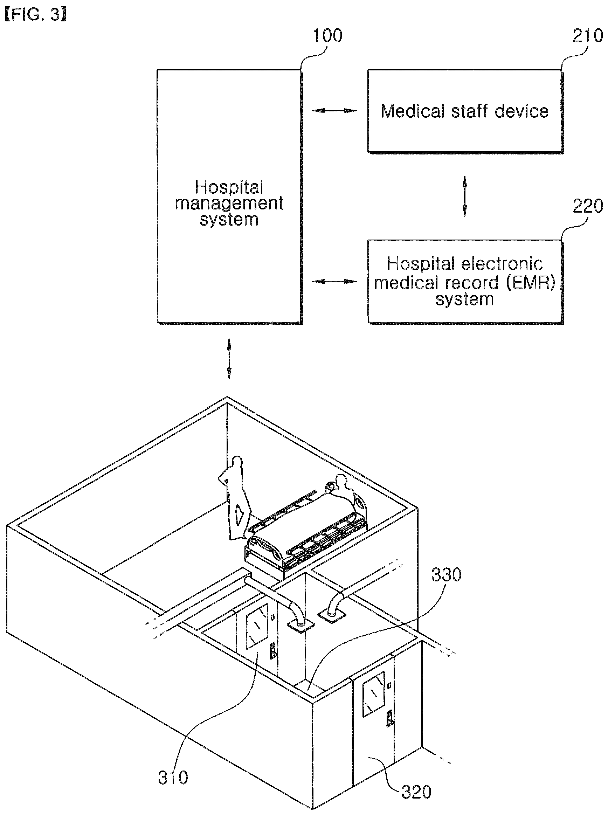

FIG. 3 is a view showing an example of diagnosis and prescription of a patient according to an embodiment of the inventive concept.

As described with reference to FIG. 2, the doctor may check the patient's biometrics and disease symptom variation through the medical staff device 210 and may transmit the prescription information to the hospital management system 100. Generally, when the doctor makes the prescription, the medical staff (e.g., the doctor who makes the prescription, or another doctor or a nurse) takes a medical action (e.g., drug administration) corresponding to the prescription. Therefore, the medical staff carries the drug to the patient room in order to carry out the medical treatment corresponding to the prescription. In this case, as described with reference to FIG. 2, the hospital management system 100 may remotely control first and second entrance doors 310 and 320 of the patient room where the patient is hospitalized.

According to one embodiment of the inventive concept, the hospital management system 100 may lock the first entrance door 310 and open the second entrance door 320 when the medical staff accesses the patient room to provide the drug to the patient. Thereafter, the medical staff leaves the drug in a front room 330 and passes through the second entrance door 320 and exits the patient room. Then, the hospital management system 100 may open the first entrance door 310 and lock the second entrance door 320 at the same time. Therefore, the medical staff may diagnose and prescribe the patient in a non-contact state with the patient.

FIG. 4 is a view showing an example of diagnosis and prescription of a patient according to an embodiment of the inventive concept.

As described with reference to FIG. 2, the doctor may check the patient's biometrics and disease symptom variation through the medical staff device 210 and may transmit the prescription information to the hospital management system 100.

According to an embodiment of the inventive concept, the hospital management system 100 may request a drug delivery robot 430 to deliver a specific drug according to the transmitted prescription information to a patient 410.

According to an embodiment of the inventive concept, when an AI speaker 420 is installed in the patient room, the AI speaker 420 may be connected to the hospital management system 100 over a network. Therefore, the hospital management system 100 may be able to interact with the patient 410 through the AI speaker 420. For example, when the drug delivery robot 430 delivers the drug to the patient or the medical staff delivers the drug in a non-contact manner, as illustrated in FIG. 3, the hospital management system 100 may provide an instruction for administering the drug to the patient through the AI speaker 420. Moreover, although not shown in FIG. 3, the AI speaker 420 may be connected to the medical staff device 210 over the network. Therefore, the medical staff may provide the instruction for administering the drug to the patient directly through the AI speaker 420, without going through the hospital management system 100.

According to one embodiment of the inventive concept, the patient 410 may utter his/her body condition. The uttered voice information may be transmitted to the hospital management system 100 through the AI speaker 420. When the voice information is transmitted, the hospital management system 100 may determine body information corresponding to the patient's voice information, and use the body information as the biometrics, and transmit the biometrics to the medical staff device 210 and the hospital electronic medical record system 220. For example, when the patient utters "I have an ache at a lower stomach", the voice information "I have an ache at a lower stomachache" may be transmitted to the hospital management system 100 through the AI speaker. In this case, the hospital management system 100 may determine "lower abdominal pain" as the patient's current body information, may use the body information as the biometrics, and may transmit the biometrics to the medical staff device 210 and the hospital electronic medical record system 220.

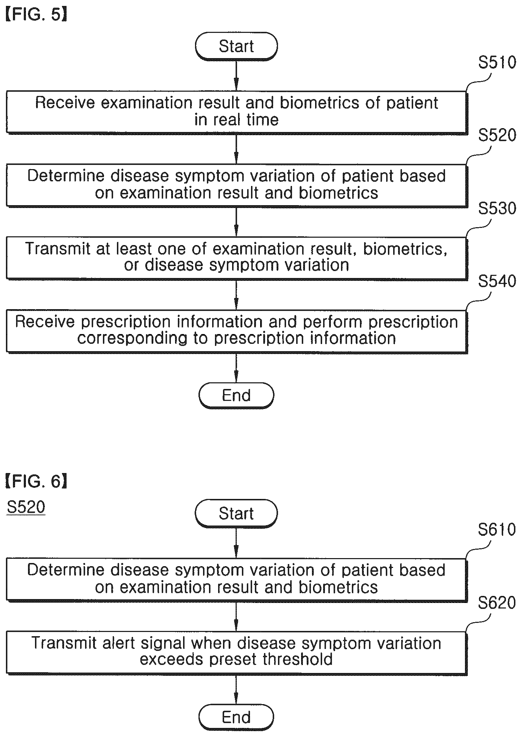

FIG. 5 is a flowchart showing a method of controlling a hospital management system according to an embodiment of the inventive concept.

In operation S510, the hospital management system 100 may receive the patient's examination results and the patient's biometrics. The examination results may be transmitted from the hospital electronic medical record system 220 thereto. After the examination result may be transmitted to the hospital management system 100, and then may be transmitted to the hospital electronic medical record system 220. Moreover, because the biometrics are real-time information corresponding to the patient's body state, the biometrics may be transmitted to the hospital management system 100 simultaneously with or in a different timing from that of the examination result.

Next, in operation S520, the hospital management system 100 may determine the disease symptom variation based on the received examination result and biometrics.

According to an embodiment of the inventive concept, the hospital management system 100 may be able to learn the examination result, the biometrics, and the disease symptom variation using the deep neural network. Therefore, the hospital management system 100 may determine the disease symptom variation and at the same time, determine whether the symptom of the current patient's disease is likely to change or whether the disease has been cured, using the deep neural network.

Next, in operation S530, the hospital management system 100 may transmit the examination result, the biometrics and the disease symptom variation at least one to the medical staff device 210 and the hospital electronic medical record system 220. Moreover, when the hospital management system 100 determines whether the symptom of the patient's disease is likely to change or whether the disease has been cured, the hospital management system 100 may transmit the determination result together with the examination result, the biometrics and the disease symptom variation.

Next, in operation S540, the hospital management system 100 may receive the prescription information from the doctor and perform the prescription corresponding to the prescription information. For example, the hospital management system 100 may request the drug delivery robot 430 to deliver the drug corresponding to the received prescription information to the patient. The hospital management system 100 may control the entrance doors 310 and 320 of the patient room so that the medical staff may deliver the drug to the patient in the non-contact manner. Moreover, the hospital management system 100 may provide the instruction for administering the drug to the patient through the display device (not shown) or the AI speaker 420 in the patient room.

FIG. 6 is a flowchart showing a method of controlling a hospital management system according to an embodiment of the inventive concept. Operation 610 of FIG. 6 may be an example of operation S520 in FIG. 5.

That is, operation S610 in FIG. 6 may correspond to operation S520 in FIG. 5, and thus detailed description thereof is omitted.

According to an embodiment of the inventive concept, the hospital management system 100 determines the patient's disease symptom variation in operation S610. When the disease symptom variation exceeds the threshold in operation S620, the hospital management system 100 may transmit the alert signal to the medical staff device 210 and the hospital electronic medical record system 220. In this case, the threshold may be pre-stored, by the medical staff, into the hospital electronic medical record system 220 or may be stored in real time. Moreover, the alert signal may be transmitted in a text form, or may be the sound information. In the latter case, when the alert signal is transmitted to the medical staff device 210, a sound occurs. Therefore, when the alert signal is transmitted, the medical staff determines that the patient is in a critical state and may perform medical actions more urgently.

Various embodiments of the inventive concept may be implemented using software including at least one instructions stored in a storage medium such as a memory which may be readable by a machine, for example, the hospital management system 100 or a computer. For example, the processor of the device, for example, the processor 130 may fetch at least one instruction among instructions stored in the storage medium, and may execute the instruction. This enables the machine to be operated to perform at least one function according to the fetched at least one instruction. The at least one instructions may include a code generated by a compiler or a code that may be executed by an interpreter. The machine-readable storage medium may be provided in a form of a non-transitory storage medium. In this connection, the term 'non-transitory storage medium' refers to a real tangible object but may not include a signal such as an electromagnetic wave. The term does not distinguish between a case where data is stored semi-permanently on the storage medium and a case where data is temporarily stored therein. For example, the term 'non-transitory storage medium' may include a buffer in which data is temporarily stored.

According to an embodiment, a method according to various embodiments disclosed in the present disclosure may be implemented in a computer program product manner. The computer program product may be traded between sellers and buyers. The computer program product may be distributed in a form of a storage medium readable by the machine, for example, a compact disc read only memory (CD-ROM). Alternatively, the computer program product may be directly distributed on-line (e.g., downloaded or uploaded) between two user devices such as smartphones through an application store such as Play Store™. In the online distribution, at least a portion of the computer program product (e.g., downloadable app) may be temporarily stored in a machine-readable storage medium such as a memory of a manufacturer server, an application store server, or a relay server or may be temporarily created.

According to the embodiments of the inventive concept, first, the medical staff may check in real time the symptom of the patient according to the disease which the patient suffers from.

According to the embodiments of the inventive concept, second, the medical staff may be able to diagnose, prescribe and treat the patient in the non-contact manner or remotely.

According to the embodiments of the inventive concept, third, the hospital management system may automatically determine the likelihood of the symptom change of a disease of the patient, the disease confirmation, and whether the patient's disease has been cured, using the deep learning algorithm through the deep neural network.

The effects of the inventive concept are not limited to the effects mentioned above. Other effects not mentioned will be clearly understood by those skilled in the art from the above descriptions.

While the inventive concept has been described with reference to exemplary embodiments, it will be apparent to those skilled in the art that various changes and modifications may be made without departing from the spirit and scope of the inventive concept. Therefore, it should be understood that the above embodiments are not limiting, but illustrative.

## Claims

1. A hospital management system comprising:
a communication unit for transmitting and receiving data;
a memory for storing at least one instruction; and
at least one processor for executing the at least one instruction stored in the memory, wherein the at least one processor is configured to:
control the communication unit to receive biometrics of a patient in real time;
determine a disease symptom variation of the patient based on an examination result and the biometrics, wherein the examination result includes at least one of CT, Xray, MRI results, a body temperature, a pulse, a blood pressure, an oxygen saturation, an electrocardiogram, or a blood sugar level of the patient;
control the communication unit to transmit at least one of the examination result, the biometrics, or the disease symptom variation to a hospital electronic medical record system and a medical staff device; and
receive prescription information from a medical staff, and perform prescription corresponding to the prescription information for the patient.

2. The system of claim 1, wherein the biometrics includes at least one of a body temperature, a pulse, a blood pressure, an oxygen saturation, an electrocardiogram, and a blood sugar level of the patient, and body information corresponding to voice information input from the patient,
wherein at least one of the body temperature, the pulse, the blood pressure, the oxygen saturation, the electrocardiogram, or the blood sugar level among the biometrics is checked in real time by a wearable device of the patient,
wherein the at least one processor is configured to control the communication unit to receive the biometrics checked by the wearable device in real time.

3. The system of claim 1, wherein the at least one processor is configured to change a data format of the examination result and the disease symptom variation to a data format of data stored in the hospital electronic medical record system and transmit the changed data format of the examination result and the disease symptom variation to the hospital electronic medical record system.

4. The system of claim 1, wherein the disease symptom variation of the patient includes a variation of the biometrics corresponding to a disease of the patient,
wherein the at least one processor is configured to transmit an alert signal to the hospital electronic medical record system and the medical staff device when the disease symptom variation exceeds a preset threshold.

5. The system of claim 1, wherein the prescription information is received remotely from the medical staff device,
wherein the prescription information includes at least one of a type of a drug to be administered to the patient, a dosage of the drug, or an administration period of the drug.

6. The system of claim 1, wherein the at least one processor is configured to provide the patient with an instruction to administer a drug delivered to the patient by the medical staff in a non-contact manner or based on remote prescription.

7. The system of claim 1, wherein the examination result, the biometrics, and the disease symptom variation of the patient act as training data for machine learning,
wherein the at least one processor is configured to learn the training data to determine at least one of a likelihood of symptom change of a disease of the patient, whether the disease is confirmed, or whether the disease has been cured.

8. The system of claim 1, wherein the medical staff device includes at least one of an electronic device of a nursing station, or an electronic device of doctor.

9. A method for controlling a hospital management system, the method comprising:
receiving biometrics of a patient in real time;
determining a disease symptom variation of the patient based on an examination result and the biometrics, wherein the examination result includes at least one of CT, Xray, MRI results, a body temperature, a pulse, a blood pressure, an oxygen saturation, an electrocardiogram, or a blood sugar level of the patient;
transmitting at least one of the examination result or the disease symptom variation to a hospital electronic medical record system and a medical staff device; and
receiving prescription information from a medical staff, and performing prescription corresponding to the prescription information for the patient.

10. The method of claim 9, wherein the disease symptom variation of the patient includes a variation of the biometrics corresponding to a disease of the patient,
wherein the determining of the disease symptom variation of the patient includes transmitting an alert signal to the hospital electronic medical record system and the medical staff device when the disease symptom variation exceeds a preset threshold.

## Amended claims

### Amended claims in accordance with Rule 137(2) EPC.

1. A hospital management system comprising:
a communication unit for transmitting and receiving data;
a memory for storing at least one instruction; and
at least one processor for executing the at least one instruction stored in the memory, wherein the at least one processor is configured to:
control the communication unit to receive biometrics of a patient in real time;
determine a disease symptom variation of the patient based on an examination result and the biometrics, wherein the examination result includes at least one of CT, Xray, MRI results, a body temperature, a pulse, a blood pressure, an oxygen saturation, an electrocardiogram, or a blood sugar level of the patient;
control the communication unit to transmit at least one of the examination result, the biometrics, or the disease symptom variation to a hospital electronic medical record system and a medical staff device; and
receive prescription information from a medical staff, and perform prescription corresponding to the prescription information for the patient;
wherein the disease symptom variation of the patient includes a variation of the biometrics corresponding to a disease of the patient;
**characterized in that**
the at least one processor further is configured
- to transmit an alert signal to the hospital electronic medical record system and the medical staff device when the disease symptom variation exceeds a preset threshold;
- to provide the patient with an instruction to administer a drug delivered to the patient by the medical staff in a non-contact manner or based on remote prescription; and
- to learn training data from machine learning to determine at least one of a likelihood of symptom change of a disease of the patient, whether the disease is confirmed, or whether the disease has been cured, the examination result, the biometrics, and the disease symptom variation of the patient acting as training data for the machine learning.

2. The system of claim 1, wherein the biometrics includes at least one of a body temperature, a pulse, a blood pressure, an oxygen saturation, an electrocardiogram, and a blood sugar level of the patient, and body information corresponding to voice information input from the patient,
wherein at least one of the body temperature, the pulse, the blood pressure, the oxygen saturation, the electrocardiogram, or the blood sugar level among the biometrics is checked in real time by a wearable device of the patient,
wherein the at least one processor is configured to control the communication unit to receive the biometrics checked by the wearable device in real time.

3. The system of claim 1, wherein the at least one processor is configured to change a data format of the examination result and the disease symptom variation to a data format of data stored in the hospital electronic medical record system and transmit the changed data format of the examination result and the disease symptom variation to the hospital electronic medical record system.

4. The system of claim 1, wherein the prescription information is received remotely from the medical staff device,
wherein the prescription information includes at least one of a type of a drug to be administered to the patient, a dosage of the drug, or an administration period of the drug.

5. The system of claim 1, wherein the medical staff device includes at least one of an electronic device of a nursing station, or an electronic device of doctor.

6. A method for controlling the hospital management system of one of claims 1 to 5, the method comprising:
receiving the biometrics of a patient in real time.
